# EUROPÄISCHE PATENTSCHRIFT

(11) **EP 2 315 608 B1**
(45) Veröffentlichungstag und Bekanntmachung des Hinweises auf die Patenterteilung: **28.11.2012**
(21) Anmeldenummer: 09777829.4
(22) Anmeldetag: 12.08.2009
(51) Int. Cl.: A61M 1/00

(54) **SAUGVORRICHTUNG ZUM ABSAUGEN VON FLÜSSIGKEIT WÄHREND EINES CHIRURGISCHEN EINGRIFFS**
SUCTION APPARATUS FOR EXTRACTING FLUID DURING A SURGICAL INTERVENTION
DISPOSITIF D'ASPIRATION POUR L'ASPIRATION DE LIQUIDES PENDANT UNE INTERVENTION CHIRURGICALE

(30) Priorität: 13.08.2008 DE 102008038908
(43) Veröffentlichungstag der Anmeldung: 04.05.2011
(73) Patentinhaber: Ruprecht-Karls-Universität Heidelberg, 69117 Heidelberg (DE)
(72) Erfinder: GLASER, Simon, 69214 Eppelheim (DE); FEDERSPIL, Philippe, 66424 Homburg (Saar) (DE); PLINKERT, Peter, K., 69120 Heidelberg (DE)
(74) Vertreter: Reiser & Partner
(86) Internationale Anmeldenummer: PCT/EP2009/005844
(87) Internationale Veröffentlichungsnummer: WO 2010/017971

(56) Entgegenhaltungen:
- WO-A1-2005/021066
- DE-B1- 2 324 415
- US-A- 4 562 838
- US-A- 5 197 963
- US-A1- 2004 172 114
- US-A1- 2005 119 648
- US-A1- 2006 200 219

## Beschreibung

Die vorliegende Erfindung betrifft eine Saugvorrichtung zum Absaugen von Flüssigkeit, insbesondere von Körperflüssigkeit, während eines chirurgischen Eingriffs, mit einer Einrichtung zur Erzeugung eines Saugdruckes und einer Absaugleitung, welche mit ihrem ersten Endabschnitt mit der Einrichtung zur Erzeugung des Saugdruckes verbunden ist und welche an ihrem zweiten Endabschnitt mit einem rohrförmigen Saugstutzen versehen ist, um Flüssigkeit aufzunehmen und durch die Absaugleitung abzuführen. Darüber hinaus betrifft die Erfindung einen Saugstutzen für eine solche Saugvorrichtung.

Saugvorrichtungen werden bei chirurgischen Eingriffen regelmäßig verwendet, um Flüssigkeiten, z.B. austretendes Blut, während der Operation aus der Wunde zu entfernen. Eine solche Vorrichtung kann insbesondere als Wundsekretsauger eingesetzt werden. An dem körperseitigen Ende der Absaugleitung ist dabei üblicherweise ein Saugstutzen vorgesehen, dessen Form sich nach dem jeweiligen Anwendungszweck richtet. Derartige Saugstutzen sind u.a. unter der Bezeichnung PLESTER-Saugkanüle oder BELUCCI-Saugrohr mit oder ohne Saugunterbrecher bekannt.

Weiterhin ist es bekannt, während eines chirurgischen Eingriffs ein neurophysiologische Monitoring durchzuführen. Hierdurch ist es möglich, nervale Strukturen des Körpers während der Operation zu überwachen und auf diese Weise Schädigungen der Nerven durch den Eingriff zu vermeiden. Auf diese Weise kann insbesondere bei mikrochirurgischen Operationstechniken der Funktionserhalt motorischer Nerven deutlich verbessert werden, um operativ bedingte Läsionen zu vermeiden oder zu reduzieren. Das intraoperative neurophysiologische Monitoring empfiehlt sich insbesondere für die Schilddrüsen- und Nebenschilddrüsenchirurgie, die Neurochirurgie, die Kopf- und Halschirurgie, wie beispielsweise Mastoidektomie, sowie für die Herz- oder Thoraxchirurgie, kann aber auch bei anderen Operationen angewandt werden.

Das intraoperative neurophysiologische Monitoring funktioniert nach folgendem Prinzip: Zunächst erfolgt eine direkte Stimulation eines Nervens mittels einer Sonde durch einen kleinen Stromstoß von beispielsweise 0,2 mA bis 2 mA. Dies führt bei intakten Nerven zu einer Muskelkontraktion, welche mittels Ableitungselektroden anhand der dabei auftretenden elektrischen Potenziale identifiziert, elektronisch verstärkt und akustisch über einen Lautsprecher und oder optisch auf einer Anzeige dargestellt werden kann. Für die Stimulation werden entweder bipolare Stimulationssonden, beispielsweise Gabelsonden, Kugelsonden, oder aber monokulare Sonden verwendet. Die vorbekannten Stimulationssonden sind jedoch separate Geräte, welche unabhängig von der Saugvorrichtung eingesetzt werden.

Aus der DE 23 24 415 ist ein chirurgisches Sauginstrument bekannt, bei dem an der Saugöffnung des Saugrohres eine Koagulationselektrode angeordnet ist, die mit einer elektrischen Leitung verbunden ist.

In der US 2005/0119648 A1 ist eine Vorrichtung gezeigt, welche geeignet ist, Gewebe zu stimulieren. Zur Stimulation des Gewebes wird diese Vorrichtung an das Gewebe herangeführt und auf das Gewebe aufgesetzt, wobei eine Saugvorrichtung die Vorrichtung auf dem Gewebe fixiert.

Aus der US 5,197,963 ist ein chirurgisches Instrument für die Elektrochirurgie bekannt, welches als elektrisches Skalpell ausgebildet ist.

Aufgabe der Erfindung ist es, eine Saugvorrichtung und einen Saugstutzen für eine solche Vorrichtung derart weiterzubilden, dass während des operativen Eingriffs eine bessere Überwachung der Nerven erfolgen kann.

Diese Aufgabe wird bei einer eingangs genannten Saugvorrichtung durch eine Einrichtung zur Nervenstimulation mit wenigstens einer Elektrode, durch die eine Nervenbahn elektrisch stimulierbar ist, und mit einer Ableitungselektrode zur Erfassung der dadurch herbeigeführten Muskelkontraktion gelöst. Die erfindungsgemäße saug vorrichtung ist in Anspruch 1 definiert.

Durch die erfindungsgemäße Ausgestaltung des Saugers wird eine verbesserte Überwachung der Nerven erreicht. Indem an dem Saugstutzen eine Einrichtung zur Nervenstimulation vorgesehen ist, kann mit dem Saugstutzen während des Eingriffs ein Absaugen von Flüssigkeit und eine Stimulation der Nerven durchgeführt werden, ohne dass hierzu ein Instrumentenwechsel erforderlich wäre. Dadurch kann die Überwachung schneller und häufiger durchgeführt werden, als dies früher der Fall war. Zudem ermöglicht die Erfindung, die Sicht auf das Operationsfeld zu verbessern, da zum einen die Anzahl der in dem Operationsfeld befindlichen Instrumente reduziert wird und zum anderen auch während der Stimulation Flüssigkeit, z.B. Blut, aufgenommen werden kann. Weiterhin kann durch eine Reduktion von Wundsekret oder anderen Flüssigkeiten während der Stimulation ein optimales Umfeld zur Nervenerregung geschaffen werden. Auch dies trägt zu einer Reduktion von Nervenverletzungen bei. Die erfindungsgemäße Saugvorrichtung kann bei allen Operationen eingesetzt, bei denen eine Überwachung der Nerven erfolgen soll. Insbesondere eignet sich die Saugvorrichtung für Schilddrüsenoperationen, Parotisoperationen, Mastoidoperationen oder neurochirurgische Operationen. Der Saugstutzen kann dabei z.B. als Saugrohr oder als ein anderer Körper ausgebildet sein, der eine mit der Absaugleitung in Wirkverbindung stehende Saugöffnung aufweist. Der Saugstutzen kann als separates Teil oder einstückig mit der Absaugleitung ausgebildet sein. Dabei ist es auch möglich, dass der Saugstutzen durch einen Endbereich der Absaugleitung gebildet wird. Die Absaugleitung kann z.B. ganz oder teilweise flexibel ausgebildet sein, wodurch der Absaugstutzen leicht in die gewünschte Position gebracht werden kann. Bei der elektrischen Stimulation über die Elektrode können Gleichströme, gepulste Gleichströme und auch Wechselströme verwendet werden, die vorzugsweise nur für kurze Zeit angelegt werden. Hierbei sind meist schon geringe Stromstärken von zwischen 0,05 mA bis 5 mA, vorzugsweise 0,2 mA bis 2 mA ausreichend, um bei einem intakten Nerv eine messbare Stimulation zu erreichen.

Nach einer vorteilhaften Ausgestaltung der Erfindung ist vorgesehen, dass die wenigstens eine Elektrode durch wenigstens eine an oder in dem Saugstutzen angeordnete elektrisch leitende Kontaktfläche gebildet ist. Auf diese Weise kann eine wirksame Stimulation erfolgen, unabhängig davon, ob währenddessen eine Aufnahme von Flüssigkeit durch den Saugstutzen erfolgt.

Eine besonders gute Stimulation lässt sich ohne Verschlechterung der Saugleistung dann erreichen, wenn die wenigstens eine Kontaktfläche ringförmig oder teilringförmig ausgebildet ist. Das gleiche gilt insbesondere dann, wenn die Kontaktfläche an dem Außenumfang des Saugstutzens angeordnet ist.

Eine weitere Verbesserung der Stimulation bei guter Saugleistung ergibt sich dann, wenn die Kontaktfläche circular oder hemicircular ausgebildet ist.

Eine besonders punktgenaue und wirksame Stimulation der Nerven wird dann erreicht, wenn die wenigstens eine Kontaktfläche eine oder mehrere von dem Saugstutzen vorstehende Stifte aufweist. Insbesondere können die Stifte Gabelförmig nebeneinander angeordnet sein. Die Enden der Stifte können spitz zulaufen oder abgerundet ausgebildet sein.

Eine weitere Verbesserung wird dadurch erreicht, dass der rohrförmige Saugstutzen einen elektrisch leitenden Endabschnitt, insbesondere aus Metall aufweist, welcher die Elektrode bildet. Dabei ist es möglich nur den elektrisch leitenden Endabschnitt oder aber den gesamten Saugstutzen aus elektrisch leitendem Material, wie z.B. Metall, zu fertigen.

Weiterhin kann erfindungsgemäß vorgesehen sein, wenigstens zwei Kontaktflächen vorgesehen sind, die insbesondere gegeneinander isoliert sind.

Nach einer besonders vorteilhaften Ausgestaltung der Erfindung ist vorgesehen, dass die wenigstens eine Elektrode an dem Saugstutzen verstellbar, insbesondere teleskopierbar angeordnet ist. Hierdurch kann die Elektrode jeweils in die gewünschte Position gebracht werden und auch bei unterschiedlichen räumlichen Gegebenheiten eine wirkungsvolle Stimulation bei guten Saugeigenschaften erreicht werden.

In vorteilhafter Weise können Energieversorgungsmittel vorgesehen sein, durch die die wenigstens eine Elektrode mit einer elektrischen Spannung beaufschlagbar ist. Die Energieversorgungsmittel können z.B. durch einen Energiespeicher und/oder wenigstens eine Schnittstelle für eine externe Energieversorgung gebildet werden.

Hierzu können die Energieversorgungsmittel eine an oder in dem Saugstutzen angeordnete elektrische Leitung umfassen, die mit der wenigstens einen Elektrode verbunden sind. Diese elektrische Leitung kann je nach Anwendungsfall ein oder mehradrig ausgebildet sein.

Zur einfachen Handhabung während der Operation können die Energieversorgungsmittel einen insbesondere an dem Saugstutzen angeordneten Schalter aufweisen, um die wenigstens eine Elektrode mit der elektrischen Spannung zu versorgen. Auf diese Weise kann die Stimulation durch den Operateur durch einen Tastendruck per Hand ausgelöst werden. Alternativ kann natürlich eine Stimulation auch durch einen Fußschalter oder einen anderen Schalter betätigt werden.

Eine einfache Reinigung und Sterilisation ergibt sich dann, wenn der Saugstutzen mit der wenigstens einen Elektrode abnehmbar ist.

Weiterhin kann erfindungsgemäß vorgesehen sein, dass die Elektrode als monopolare oder bipolare Elektrode ausgebildet ist. Die bipolare Ausgestaltung hat dabei den Vorteil, dass die Kalibrierung der Elektrode einfacher ist. Monopolare Elektroden müssen hingegen meist manuell kalibriert werden.

Die Einrichtung zur Erzeugung des Saugdrucks kann vorteilhafter Weise eine elektrisch und/oder mechanisch angetriebene Einrichtung, z.B. eine Pumpe sein. Natürlich ist es auch möglich, eine ohnehin im Operationssaal vorhandene Unterdruckleitung als Einrichtung zur Erzeugung eines Saugdruckes einzusetzen. Auf diese Weise können z.B. Blut, Wundsekret, Verdauungsprodukte etc. problemlos abgesaugt werden.

Die Gefahr von Verletzungen während des Saugvorgangs kann dann vermindert werden, wenn in dem Saugstutzen wenigstens eine Bypassöffnung ausgebildet ist. Eine solche Bypassöffnung, welche benachbart zur Saugöffnung angeordnet sein kann, ermöglicht ein atraumatisches Saugen.

Eine Steuerung des Saugdruckes kann auf einfache Weise erreicht werden, wenn der Saugstutzen wenigstens eine Regulationsöffnung aufweist. Auf diese Weise kann durch Öffnen und Schließen der Regulationsöffnung, beispielsweise durch Auflegen eines Fingers, die Saugkraft auf einfache Weise angepasst werden.

Ein Saugstutzen für die vorbeschriebene Saugvorrichtung umfasst einen rohrförmigen Abschnitt, wobei an dem rohrförmigen Abschnitt eine Einrichtung zur Nervenstimulation mit wenigstens einer Elektrode zum elektrischen Stimulieren einer Nervenbahn während einer chirurgischen Operation angeordnet ist.

Weitere Ziele, Merkmale, Vorteile und Anwendungsmöglichkeiten der vorliegenden Erfindung ergeben sich aus der nachfolgenden Beschreibung von Ausführungsbeispielen anhand der Zeichnungen. Dabei bilden alle beschriebenen und/oder bildlich dargestellten Merkmale für sich oder in beliebiger Kombination den Gegenstand der Erfindung, auch unabhängig von der Zusammenfassung in einzelnen Ansprüchen oder deren Rückbeziehung.

Es zeigen:
- Fig. 1: eine schematische Darstellung einer erfindungsgemäßen Saugvorrichtung
- Fig. 2: ein Blockschaltbild einer erfindungsgemäßen Saugvorrichtung mit einem Systems zum intraoperativen Neuromonitoring;
- Fig. 3: eine erste Ausführungsvariante des Saugstutzens einer erfindungsgemäßen Saugvorrichtung;
- Fig. 4: eine zweite Ausführungsvariante des Saugstutzens einer erfindungsgemäßen Saugvorrichtung;
- Fig. 5: eine dritte Ausführungsvariante des Saugstutzens einer erfindungsgemäßen Saugvorrichtung;
- Fig. 6: eine vierte Ausführungsvariante des Saugstutzens einer erfindungsgemäßen Saugvorrichtung;
- Fig. 7: eine fünfte Ausführungsvariante des Saugstutzens einer erfindungsgemäßen Saugvorrichtung und
- Fig. 8: eine Teildarstellung einer weiteren Ausführungsvariante eines Saugstutzens einer erfindungsgemäßen Saugvorrichtung mit Regulationsöffnungen.

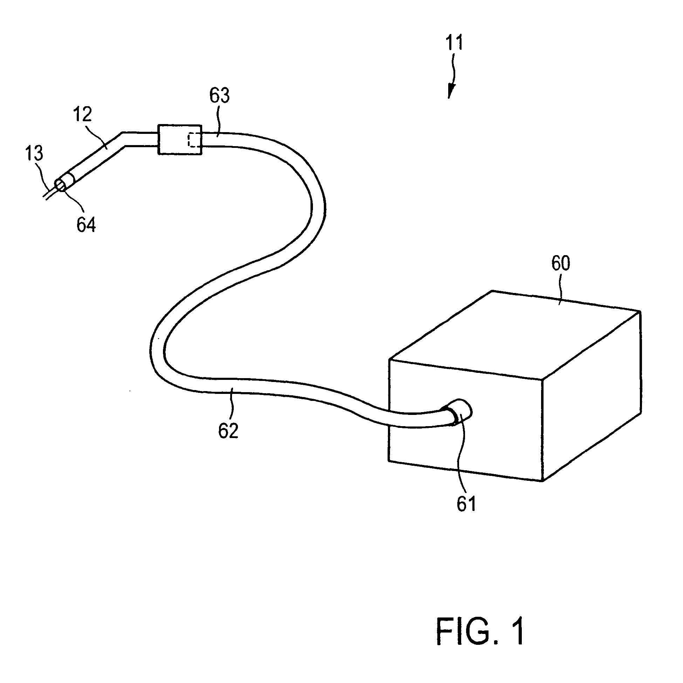

Figur 1 zeigt eine schematische Darstellung einer erfindungsgemäßen Saugvorrichtung 11 zum Absaugen von Flüssigkeit, insbesondere Körperflüssigkeit, während eines chirurgischen Eingriffs. Die Saugvorrichtung 11 weist eine Einrichtung 60 zur Erzeugung eines Saugdruckes auf, die bei der dargestellten Ausführungsform als elektrische Unterdruckpumpe ausgebildet ist. Alternativ kann der Saugdruck auch auf andere Weise bereitgestellt werden. Sofern im Operationssaal eine Unterdruckleitung zur Verfügung steht, kann diese zur Erzeugung des Saugdruckes eingesetzt werden. An die Einrichtung 60 ist über einen ersten Endabschnitt 61 eine Absaugleitung 62 angeschlossen. Die Absaugleitung 62 ist bei der dargestellten Ausführungsform beweglich, z.B. als flexibler Schlauch aus Kunststoff, ausgebildet, sodass der zweite Endabschnitt 63 leicht zu dem Operationsfeld bewegt werden kann. An dem zweiten Endabschnitt 63 ist ein rohrförmiger Saugstutzen 12 angeordnet, der eine Saugöffnung 64 aufweist, mit der unter Wirkung des Saugdruckes Flüssigkeit aufgesaugt werden kann. Der Saugstutzen 12 ist mit der Absaugleitung 62 lösbar verbunden und kann zum Reinigen und Sterilisieren von dieser abgenommen werden. Der Saugstutzen kann aber auch, anders als dargestellt, einstückig mit der Absaugleitung 62 ausgebildet sein und/oder durch den zweiten Endabschnitt 63 gebildet werden. Die Saugvorrichtung 11 weist zudem an dem Saugstutzen 12 angeordnete Elektroden 13 auf, welche Teil einer unten näher beschriebenen Einrichtung zur Nervenstimulation sind. Der rohrförmige Saugstutzen kann ganz oder teilweise aus einem starren Material gebildet sein. Alternativ kann der rohrförmige Saugstutzen 12 ganz oder teilweise aus einem verformbaren Material, z.B. als Schlauch ausgestaltet sein.

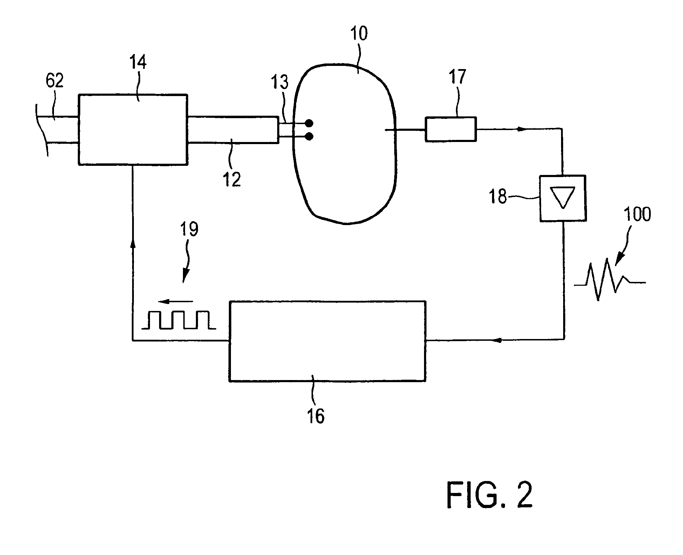

Figur 2 zeigt in einem Blockschaltbild wie die Einrichtung zur Nervenstimulation 16 ausgebildet und angeordnet ist, welche eine intraoperative Überwachung von Nerven ermöglicht. Hier ist zunächst ein zu überwachendes Körperteil 10, beispielsweise eine Schilddrüse, dargestellt. Weiterhin ist schematisch der Saugstutzen 12 erkennbar. Während einer Operation kann über den durch die Einrichtung zur Erzeugung eines Saugdruckes erzeugten Unterdruck Flüssigkeit, z.B. Blut, von dem Saugstutzen 12 aufgenommen und über die Absaugleitung 62 zu einem nicht dargestellten Auffangbehälter transportiert werden. Hierzu ist eine Steuerung 14 für das Absaugen vorgesehen. Mittels eines an dieser Steuerung 14 vorgesehenen Bedienelements (nicht dargestellt) kann der Operateur das Absaugen initiieren.

Die Einrichtung zur Nervenstimulation 16 weist wenigstens eine an dem Saugstutzen 12 angeordnete Elektrode 13 auf. Die Einrichtung zur Nervenstimulation 16 weist zudem Energieversorgungsmittel auf, welche elektrische Impulse 19 erzeugt, die über eine in oder an der Absaugleitung 62 angeordnete elektrische Leitung zu den Elektroden 13 weitergeleitet werden. Durch diese elektrischen Impulse 19 werden Nerven am Körperteil 10 stimuliert, sobald die Elektrode an den zu überwachenden Nerv angelegt wird. Hierfür ist in der Regel ein Stromstoß von beispielsweise zwischen 0,2 mA und 2 mA ausreichend. Die elektrische Stimulation führt bei einer intakten Nervenbahn zu Muskelkontraktionen, welche über entsprechende Ableitungselektroden erfasst und in elektrische Signale umgewandelt werden können. Hierzu ist als Ableitungselektrode eine Sonde 17 vorgesehen, welche als Einstechelektrode ausgebildet ist und in den zu überwachenden Muskel eingestochen werden kann. Durch ein Eindringen der Sonde 17 in den Muskel wird eine genaue Messung ermöglicht. Alternativ kann eine elektrische Ableitung aber auch durch andere ausgestaltete Ableitungselektroden erfolgen. Das auf diese Weise erzeugte Signal wird dann mittels des Verstärkers 18 vorverstärkt, so dass ein Signal 100 zur Weiterverarbeitung zur Verfügung steht. In Abhängigkeit von der Analyse dieses Signals 100 erfolgt eine Ausgabe, ob die Nerven noch auf die vorgenommene Stimulation reagieren. Diese Ausgabe kann akustisch und/oder über eine Anzeige erfolgen. Damit ist es dem Operateur möglich, die Auswirkungen seiner Eingriffe bereits frühzeitig zu erkennen.

Die Einrichtung zur Nervenstimulation 16 weist für die Analyse beispielsweise einen Prozessor auf, um das Signal 100 zu verarbeiten und ein entsprechendes Ausgabesignal für die akustische und/oder optische und/oder haptische Ausgabe zu erzeugen. Weiterhin ist ein Impulsgenerator vorgesehen, um die elektrischen Impulse 19 zu erzeugen. Auch eine akustische Ausgabe sowie eine Anzeige kann die Einrichtung zur Nervenstimulation 16 bereits enthalten oder ggf. solche Einrichtungen ansteuern.

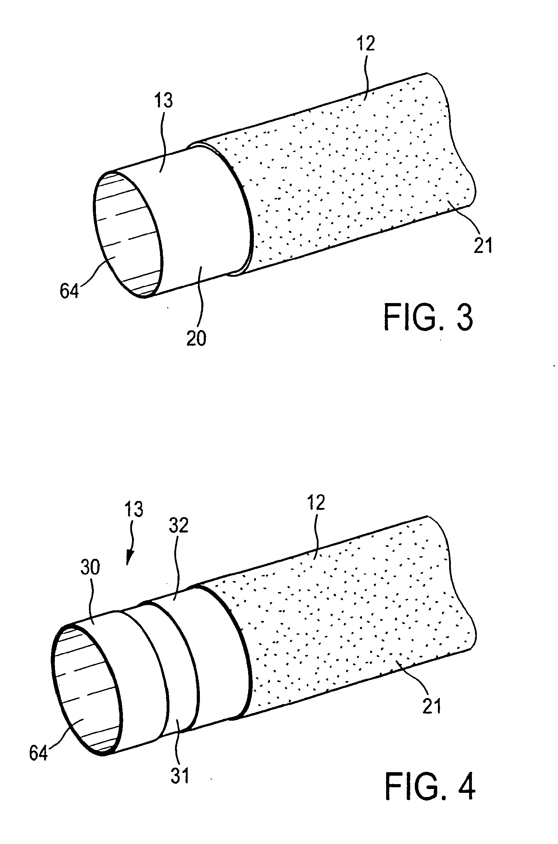

Die Figur 3 bis 7 zeigen fünf verschiedene Ausführungsvarianten eines erfindungsgemäßen Saugstutzens 12. Dargestellt ist jeweils das körperseitige Ende des rohrförmigen Saugstutzens 12 in Teilansicht. In den Figuren wird jeweils auf Teile gleicher Funktion mit denselben Bezugszeichen verwiesen.

Figur 3 zeigt eine Elektrode 13, welche durch eine an dem Saugstutzen 12 angeordnete Kontaktfläche 20 gebildet wird. Die Kontaktfläche 20 ist ringförmig ausgebildet und an dem Ende des rohrförmigen Saugstutzens 12 angeordnet. Auf diese Weise wird eine monopolare Elektrode 13 gebildet. Die Kontaktfläche 20 wird durch ein elektrisch leitendes Material, z.B. Metall, gebildet und kann über eine nicht dargestellte elektrische Leitung mit dem Impuls 19 beaufschlagt werden. Die ringförmige Kontaktfläche ist dabei koaxial an dem rohrförmigen Abschnitt 21 des Saugstutzens angeordnet und bildet einen Endabschnitt des Saugstutzens 12 mit der Saugöffnung 64. Die Kontaktfläche 20 ist derart an dem Saugstutzen 12 vorgesehen, dass aufgenommene Flüssigkeit durch den von der ringförmigen Kontaktfläche begrenzten Ringraum aufgenommen werden kann. Der rohrförmige Abschnitt 21 kann zudem mit einer Isolierschicht 21, z.B. aus Kunststoff umgeben sein. Die Isolierschicht 21 kann beispielsweise schlauchförmig ausgebildet sein.

Bei der in Figur 4 dargestellten Ausführungsform ist eine bipolare Elektrode 13 vorgesehen, welche zwei nebeneinander angeordnete ringförmige Kontaktflächen 30, 32 aufweist. Zwischen den beiden koaxial angeordneten Kontaktflächen 30, 31 ist ein isolierender Bereich, der hier als Isolierring 31 ausgebildet ist, vorgesehen. Die Kontaktflächen 30, 31 sind beide circular ausgebildet und haben einen übereinstimmenden Durchmesser. Die Kontaktflächen 30 und 32 sind dabei an getrennte und voneinander elektrisch isolierte Leitungen angeschlossen und können einen Plus- und Minuspol der Elektrode 13 bilden.

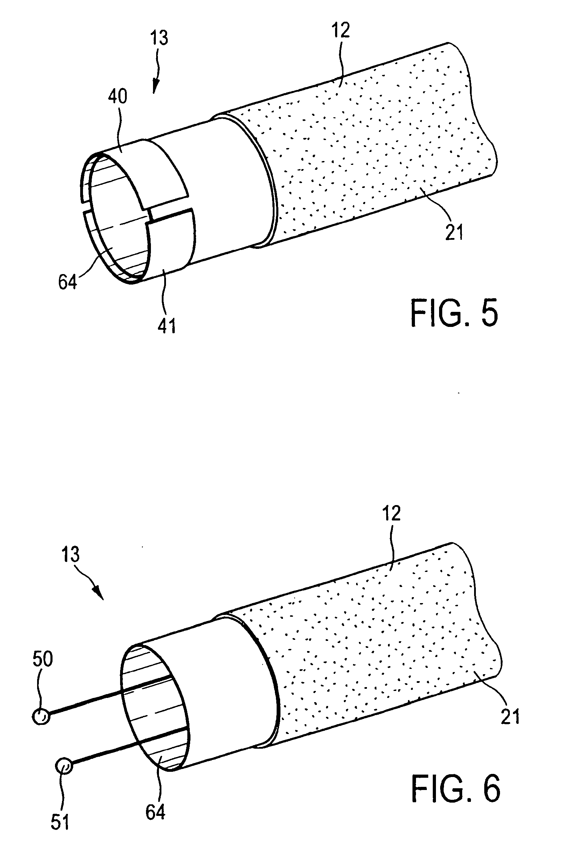

Bei der in Figur 5 dargestellten Ausführungsform des Saugstutzens 12 sind für die Elektroden 13 zwei teilringförrnige Kontaktflächen 40, 41 vorgesehen. Diese sind hemicircular ausgebildet und in Umfangsrichtung durch einen Luftspalt voneinander getrennt. Alternativ kann auch ein isolierendes Material zwischen den beiden Kontaktflächen vorgesehen sein. Die Kontaktflächen 40, 41 sind gegeneinander elektrisch isoliert, bilden eine bipolare Elektrode 13 und sind an nicht dargestellte Leitungen angeschlossen, um die Impulse 19 für die Nervenstimulation bis zu den Kontaktflächen 40, 41 zu leiten.

Bei der in Figur 6 dargestellten Ausführungsform weist die Elektrode 13 Stifte 50, 51 auf, welche die Kontaktflächen bilden. Bei der dargestellten Ausführungsform sind zwei Stifte 50, 51 vorgesehen, die gabelförmig ausgebildet und parallel nebeneinander angeordnet sind. Die Stifte 50, 51 stehen von dem Saugstutzen 12 vor und sind in dem Inneren des rohrförmigen Saugstutzens 12 befestigt. Sie erstrecken sich aus der Saugöffnung 64 heraus und stehen über den rohrförmigen Rand des Saugstutzens 12 vor. Die hier als Stifte 50, 51 ausgebildete Elektrode 13 kann verstellbar an dem Saugstutzen angeordnet sein, so dass sie eine mehr oder weniger vorstehende Position zu dem rohrförmigen Abschnitt 21 einnimmt. Zum einfachen Verstellen der Elektrode 13 kann ein nicht dargestelltes Bedienelement, welches z.B. als Schieber ausgebildet sein kann, vorgesehen werden. Bei der dargstellten Ausführungsform sind die Stifte an ihrem äußeren Ende abgerundet und weisen hierzu einen erweiterten, insbesondere kugeligen Abschnitt auf.

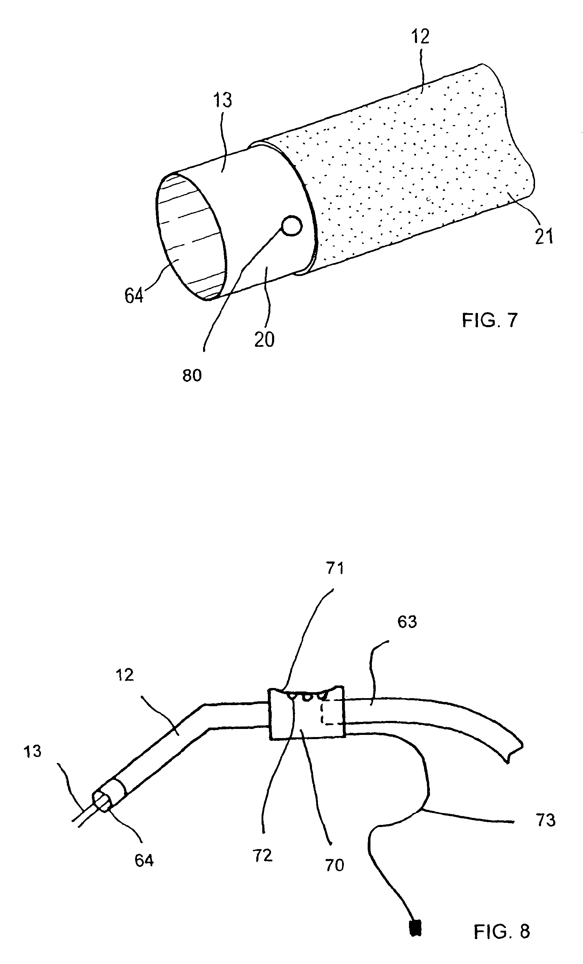

Die in Figur 7 dargestellte Ausführungsform zeigt einen Saugstutzen 12 mit einer Bypassöffnung 80. Die Bypassöffnung 80 ist in der Wandung des Saugstutzens 12 benachbart zur Saugöffnung 64 ausgebildet. Die Bypassöffnung 80 ermöglicht ein atraumatisches Saugen auch unter schwierigen Bedingungen. Wenn die Saugöffnung 64 an der Oberfläche eines Organs zu liegen kommt und dabei verschlossen wird, kann durch die Bypassöffnung 80 weiterhin Luft oder Flüssigkeit angesaugt werden. Die Bypassöffnung 80 kann vorzugsweise 2 bis 4mm von dem Rand der Saugöffnung 64 beabstandet angeordnet sein. Vorteilhafterweise können an dem Umfang des Saugstutzens 12 mehrere Bypassöffnungen 80 angeordnet sein, beispielsweise vier Bypassöffnungen, die über den Umfang verteilt sind. Die gesamte Querschnittsfläche der Bypassöffnung 80 bzw. der Bypassöffnungen 80 ist kleiner als die Querschnittsfläche der Saugöffnung 64. Die in Figur 7 gezeigt Ausführungsform entspricht im Übrigen der in Figur 3 dargestellten Ausführungsform. Die diesbezügliche Beschreibung gilt entsprechend für Figur 7.

Figur 8 zeigt eine Ausführungsform der Saugvorrichtung mit einem dem abgewinkelten Saugstutzen 12, welcher ein Griffstück 70 aufweist. Das Griffstück 70 ist mit einer Grifffläche 71 versehen, welche bei der dargestellten Ausführungsform nach innen gewölbt ist. In der Grifffläche 71 sind Regulationsöffnungen 72 vorgesehen, welche die Innenseite des Saugstutzens 12 mit der Umgebung verbinden. Indem die Regulationsöffnungen 72 während der Verwendung, beispielsweise mit dem Finger, verschlossen bzw. geöffnet werden, kann die Saugwirkung auf einfache und schnelle Weise reguliert werden. In Figur 8 ist weiterhin eine elektrische Anschlussleitung 73 dargestellt, welche mit der Elektrode 13 in Verbindung steht.

Bei allen Ausführungsformen kann der Saugstutzen 12 als Steckteil ausgebildet sein, der lösbar mit der Absaugleitung 62 verbindbar ist. Der Saugstutzen 12 mit den Kontaktflächen kann dann zum Reinigen abgenommen und z.B. durch Autoklavieren sterilisiert werden. Zudem kann in dem Saugstutzen eine nicht dargestellte Sauger-BypassÖffnung vorgesehen sein, die es dem Operateur ermöglicht, die an der Saugöffnung 64 anliegende Saugkraft manuell zu regulieren.

### Bezugszeichenliste

- 10: Körperteil
- 11: Saugvorrichtung zum Absaugen von Körperflüssigkeit
- 12: Saugstutzen
- 13: Elektrode
- 14: Steuerung für das Absaugen
- 16: Einrichtung zur Nervenstimulation
- 17: Sonde
- 18: Vorverstärker
- 19: Impulse
- 100: Signal
- 20: Kontaktfläche
- 21: rohrförmiger Abschnitt
- 30: Kontaktfläche
- 31: Isolierring
- 32: Kontaktfläche
- 40: Kontaktfläche
- 41: Kontaktfläche
- 50: Kontaktfläche
- 51: Kontaktfläche
- 60: Einrichtung zur Erzeugung eines Saugdrucks
- 61: erster Endabschnitt
- 62: Absaugleitung
- 63: zweiter Endabschnitt
- 64: Saugöffnung
- 70: Griffstück
- 71: Grifffläche
- 72: Regulationsöffnungen
- 73: Anschlussleitung
- 80: Bypassöffnung

## Patentansprüche

1. Saugvorrichtung zum Absaugen von Flüssigkeit, insbesondere Körperflüssigkeit, während eines chirurgischen Eingriffs, mit einer Einrichtung zur Erzeugung eines Saugdruckes (60) und einer Absaugleitung (62), welche mit ihrem ersten Endabschnitt (61) mit der Einrichtung zur Erzeugung des Saugdruckes (60) verbunden ist und welche an ihrem zweiten Endabschnitt (63) mit einem rohrförmigen Saugstutzen (12) versehen ist, um Flüssigkeit aufzunehmen und durch die Absaugleitung (62) abzuführen, **gekennzeichnet durch** eine Einrichtung zur Nervenstimulation (16) mit wenigstens einer Elektrode (13), zur elektrischen Stimulation einer Nervenbahn und mit einer Ableitungselektrode zur Erfassung der **dadurch** herbeigeführten Muskelkontraktion.

2. Saugvorrichtung nach Anspruch 1, **dadurch gekennzeichnet, dass** die wenigstens eine Elektrode (13) durch wenigstens eine an oder in dem Saugstutzen angeordnete elektrisch leitende Kontaktfläche (20, 30, 32, 40, 41, 50, 51) gebildet ist.

3. Saugvorrichtung nach Anspruch 2, **dadurch gekennzeichnet, dass** die wenigstens eine Kontaktfläche (20, 30, 32, 40, 41) ringförmig oder teilringförmig ausgebildet ist.

4. Saugvorrichtung nach Anspruch 2 oder 3, **dadurch gekennzeichnet, dass** die Kontaktfläche (20, 30, 32, 40, 41) an dem Außenumfang des Saugstutzen (12) angeordnet ist.

5. Saugvorrichtung nach einem der Ansprüche 2 bis 4, **dadurch gekennzeichnet, dass** die Kontaktfläche (20, 30, 32, 40, 41) circular oder hemicircular ausgebildet ist.

6. Saugvorrichtung nach einem der Ansprüche 2 bis 5, **dadurch gekennzeichnet, dass** die wenigstens eine Kontaktfläche eine oder mehrere von dem Saugstutzen (12) vorstehende Stifte (50, 51) aufweist.

7. Saugvorrichtung nach einem der Ansprüche 2 bis 6, **dadurch gekennzeichnet, dass** der rohrförmige Saugstutzen (12) einen elektrisch leitenden Endabschnitt, insbesondere aus Metall aufweist, welcher die Elektrode (13) bildet.

8. Saugvorrichtung nach einem der Anspruche 2 bis 7, **dadurch gekennzeichnet, dass** wenigstens zwei Kontaktflächen (30, 32, 40, 41, 50, 51) vorgesehen sind, die insbesondere gegeneinander isoliert sind.

9. Saugvorrichtung nach einem der Ansprüche 1 bis 8, **dadurch gekennzeichnet, dass** die wenigstens eine Elektrode (13) an dem Saugstutzen (12) verstellbar, insbesondere teleskopierbar angeordnet ist.

10. Saugvorrichtung nach einem der Ansprüche 1 bis 9, **dadurch gekennzeichnet, dass** Energieversorgungsmittel vorgesehen sind, durch die die wenigstens eine Elektrode mit einer elektrischen Spannung beaufschlagbar ist.

11. Saugvorrichtung nach Anspruch 10, **dadurch gekennzeichnet, dass** die Energieversorgungsmittel eine an oder in dem Saugstutzen angeordnete elektrische Leitung umfasst, die mit der wenigstens einen Elektrode verbunden sind.

12. Saugvorrichtung nach einem der Ansprüche 10 bis 11, **dadurch gekennzeichnet, dass** die Energieversorgungsmittel einen insbesondere an dem Saugstutzen angeordneten Schalter aufweisen, um die wenigstens eine Elektrode mit der elektrischen Spannung zu versorgen.

13. Saugvorrichtung nach einem der Ansprüche 1 bis 12, **dadurch gekennzeichnet, dass** der Saugstutzen (12) mit der wenigstens einen Elektrode (13) abnehmbar ist.

14. Saugvorrichtung nach einem der Ansprüche 1 bis 13, **dadurch gekennzeichnet, dass** die wenigstens eine Elektrode (13) als monopolare oder bipolare Elektrode ausgebildet ist.

15. Saugvorrichtung nach einem der Ansprüche 1 bis 14, **dadurch gekennzeichnet, dass** in dem Saugstutzen (12) wenigstens eine Bypassöffnung (80) ausgebildet ist.

16. Saugvorrichtung nach einem der Ansprüche 1 bis 15, **dadurch gekennzeichnet, dass** in dem Saugstutzen (12) wenigstens eine Regulationsöffnung (72) ausgebildet ist.

## Claims

1. Suction device for extracting liquid, in particular bodily fluid, by suction during a surgical intervention, comprising an apparatus for generating a suction pressure (60) and an extraction line (62) which with the first end section (61) thereof is connected to the apparatus for generating the suction pressure (60) and which at the second end section (63) thereof is provided with a pipe-shaped suction nozzle (12) in order to take up liquid and discharge it through the extraction line (62), **characterized by** an apparatus for nerve stimulation (16) comprising at least one electrode (13) for electric stimulation of a nerve tract and having a lead electrode for capturing the muscle contraction caused thereby.

2. Suction device according to Claim 1, **characterized in that** the at least one electrode (13) is formed by at least one electrically conductive contact surface (20, 30, 32, 40, 41, 50, 51) arranged on or in the suction nozzle.

3. Suction device according to Claim 2, **characterized in that** the at least one contact surface (20, 30, 32, 40, 41) has an annular or part-annular design.

4. Suction device according to Claim 2 or 3, **characterized in that** the contact surface (20, 30, 32, 40, 41) is arranged on the external circumference of the suction nozzle (12).

5. Suction device according to one of Claims 2 to 4, **characterized in that** the contact surface (20, 30, 32, 40, 41) has a circular or semicircular design.

6. Suction device according to one of Claims 2 to 5, **characterized in that** the at least one contact surface has one or more pins (50, 51) projecting from the suction nozzle (12).

7. Suction device according to one of Claims 2 to 6, **characterized in that** the pipe-shaped suction nozzle (12) has an electrically conductive end section, in particular made of metal, which forms the electrode (13).

8. Suction device according to one of Claims 2 to 7, **characterized in that** at least two contact surfaces (30, 32, 40, 41, 50, 51) are provided, which in particular are insulated from one another.

9. Suction device according to one of Claims 1 to 8, **characterized in that** the at least one electrode (13) is arranged in an adjustable, more particularly extendable, fashion on the suction nozzle (12).

10. Suction device according to one of Claims 1 to 9, **characterized in that** provision is made for energy supply means, by means of which an electric voltage can be applied to the at least one electrode.

11. Suction device according to Claim 10, **characterized in that** the energy supply means comprises an electric lead arranged on or in the suction nozzle, said electric lead being connected to the at least one electrode.

12. Suction device according to one of Claims 10 to 11, **characterized in that** the energy supply means have a switch, arranged on the suction nozzle in particular, in order to supply the at least one electrode with the electric voltage.

13. Suction device according to one of Claims 1 to 12, **characterized in that** the suction nozzle (12) with the at least one electrode (13) can be removed.

14. Suction device according to one of Claims 1 to 13, **characterized in that** the at least one electrode (13) is embodied as monopolar or bipolar electrode.

15. Suction device according to one of Claims 1 to 14, **characterized in that** at least one bypass opening (80) is formed in the suction nozzle (12).

16. Suction device according to one of Claims 1 to 15, **characterized in that** at least one regulation opening (72) is formed in the suction nozzle (12).

## Revendications

1. Dispositif d'aspiration pour l'aspiration de liquides, notamment de liquides corporels, pendant une intervention chirurgicale, comprenant un système pour produire une pression d'aspiration (60) et une conduite d'aspiration (62) qui est connectée par sa première portion d'extrémité (61) au système pour produire la pression d'aspiration (60) et qui est pourvue à sa deuxième portion d'extrémité (63) d'un raccord d'aspiration tubulaire (12) afin de recevoir du liquide et de l'évacuer par la conduite d'aspiration (62), **caractérisé par** un système de stimulation nerveuse (16) comprenant au moins une électrode (13) pour la stimulation d'une voie nerveuse et au moins une électrode de dérivation pour détecter la contraction musculaire en résultant.

2. Dispositif d'aspiration selon la revendication 1, **caractérisé en ce que** l'au moins une électrode (13) est formée par au moins une surface de contact (20, 30, 32, 40, 41, 50, 51) électriquement conductrice disposée sur ou dans le raccord d'aspiration.

3. Dispositif d'aspiration selon la revendication 2, **caractérisé en ce que** l'au moins une surface de contact (20, 30, 32, 40, 41) est réalisée sous forme annulaire ou partiellement annulaire.

4. Dispositif d'aspiration selon la revendication 2 ou 3, **caractérisé en ce que** la surface de contact (20, 30, 32, 40, 41) est disposée sur la périphérie extérieure du raccord d'aspiration (12).

5. Dispositif d'aspiration selon l'une quelconque des revendications 2 à 4, **caractérisé en ce que** la surface de contact (20, 30, 32, 40, 41) est réalisée sous forme circulaire ou semi-circulaire.

6. Dispositif d'aspiration selon l'une quelconque des revendications 2 à 5, **caractérisé en ce que** l'au moins une surface de contact présente une ou plusieurs goupilles (50, 51) saillant depuis le raccord d'aspiration (12).

7. Dispositif d'aspiration selon l'une quelconque des revendications 2 à 6, **caractérisé en ce que** le raccord d'aspiration tubulaire (12) présente une portion d'extrémité électriquement conductrice, notamment en métal, qui constitue l'électrode (13).

8. Dispositif d'aspiration selon l'une quelconque des revendications 2 à 7, **caractérisé en ce qu'**au moins deux surfaces de contact (30, 32, 40, 41, 50, 51) sont prévues, lesquelles sont notamment isolées l'une de l'autre.

9. Dispositif d'aspiration selon l'une quelconque des revendications 1 à 8, **caractérisé en ce que** l'au moins une électrode (13) est disposée de manière déplaçable sur le raccord d'aspiration (12), notamment de manière télescopique.

10. Dispositif d'aspiration selon l'une quelconque des revendications 1 à 9, **caractérisé en ce que** des moyens d'alimentation en énergie sont prévus, lesquels permettent de solliciter l'au moins une électrode avec une tension électrique.

11. Dispositif d'aspiration selon la revendication 10, **caractérisé en ce que** les moyens d'alimentation en énergie comprennent une conduite électrique disposée sur ou dans le raccord d'aspiration, laquelle est connectée à l'au moins une électrode.

12. Dispositif d'aspiration selon l'une quelconque des revendications 10 ou 11, **caractérisé en ce que** les moyens d'alimentation en énergie présentent un commutateur disposé notamment sur le raccord d'aspiration afin d'alimenter l'au moins une électrode en tension électrique.

13. Dispositif d'aspiration selon l'une quelconque des revendications 1 à 12, **caractérisé en ce que** le raccord d'aspiration (12) peut être enlevé avec l'au moins une électrode (13).

14. Dispositif d'aspiration selon l'une quelconque des revendications 1 à 13, **caractérisé en ce que** l'au moins une électrode (13) est réalisée sous forme d'électrode unipolaire ou bipolaire.

15. Dispositif d'aspiration selon l'une quelconque des revendications 1 à 14, **caractérisé en ce qu'**au moins une ouverture de dérivation (80) est réalisée dans le raccord d'aspiration (12).

16. Dispositif d'aspiration selon l'une quelconque des revendications 1 à 15, **caractérisé en ce qu'**au moins une ouverture de régulation (72) est réalisée dans le raccord d'aspiration (12).
